# EUROPEAN PATENT SPECIFICATION

(11) **EP 3 431 009 B1**
(45) Date of publication and mention of the grant of the patent: **07.08.2024**
(21) Application number: 17766006.5
(22) Date of filing: 04.01.2017
(51) Int. Cl.: A61B 8/14, G06T 1/00, A61B 8/00, G06T 7/33

(54) **ULTRASONIC DIAGNOSIS DEVICE AND METHOD FOR CONTROLLING ULTRASONIC DIAGNOSIS DEVICE**
ULTRASCHALLDIAGNOSEVORRICHTUNG UND VERFAHREN ZUR STEUERUNG DER ULTRASCHALLDIAGNOSEVORRICHTUNG
DISPOSITIF DE DIAGNOSTIC PAR ULTRASONS ET PROCÉDÉ DE COMMANDE D'UN DISPOSITIF DE DIAGNOSTIC PAR ULTRASONS

(30) Priority: 14.03.2016 JP 2016049927
(43) Date of publication of application: 23.01.2019
(73) Proprietor: FUJIFILM Corporation, Tokyo 106-8620 (JP)
(72) Inventor: EBATA, Tetsurou, Ashigara-kami-gun Kanagawa 258-8538 (JP)
(74) Representative: Klunker IP Patentanwälte PartG mbB
(86) International application number: PCT/JP2017/000038
(87) International publication number: WO 2017/158998

(56) References cited:
- WO-A1-2014/034948
- JP-A- H04 224 738
- US-A1- 2010 036 248
- US-A1- 2016 022 247

## Description

### BACKGROUND OF THE INVENTION

### 1. Field of the Invention

The present invention relates to an ultrasound diagnostic apparatus and a method for controlling the ultrasound diagnostic apparatus, and more particularly, to an ultrasound diagnostic apparatus that recognizes an imaging part of a subject on the basis of an ultrasound image.

### 2. Description of the Related Art

In the related art, an ultrasound diagnostic apparatus using an ultrasound image has been put to practical use in the medical field. In general, in this type of ultrasound diagnostic apparatus, an ultrasound probe provided with an array transducer scans a subject with an ultrasound beam and receives ultrasound echoes from the subject and the received signal is electrically processed to generate an ultrasound image.

In recent years, an ultrasound diagnostic apparatus which collates a generated ultrasound image to automatically recognize an imaging part of a subject and assists the diagnosis of the subject on the basis of the recognition result has been researched and developed. For example, JP2002-140689A discloses a medical image processing apparatus which collates a generated ultrasound image with a plurality of collation patterns corresponding to a plurality of examination parts that are stored in advance to recognize an imaging part and provides auxiliary data used for the diagnosis of a subject on the basis of the recognition result.

US2016022247 discloses the preamble of claim 1. US2010/036248A1 discloses a medical image diagnostic apparatus, medical image measuring method, and medical image measuring program. An ultrasonic diagnostic apparatus acquires input image information, which is image data of a subject, and holds it in a storage section. An image selection section performs image recognition calculation for comparison between the input image information and past image information pieces of an image measurement database. When the image recognition has succeeded, a measurement-position setting section refers to a record of image measurement information including a past image information piece most similar to the input image information, retrieves measurement position information of the record, and sets it. A measurement-position setting processing section displays the measurement position on a display section such that it is superimposed on the input image information. A measurement calculation section performs measurement calculation for the measurement position set by the measurement-position setting section, and displays measurement results on the display section. The measurement-position setting processing section 6 updates the image measurement database on the basis of the input image information, the measurement position set by the measurement-position setting section.

### SUMMARY OF THE INVENTION

However, the medical image processing apparatus disclosed in JP2002-140689A calculates similarities indicating how the generated ultrasound image and a plurality of collation patterns are similar to each other and confirms that an examination part corresponding to the collation pattern with the highest similarity is the imaging part. Therefore, even in a case in which each of the similarities calculated for each collation pattern is low, the examination part corresponding to any one of the collation patterns is confirmed to be the imaging part of the subject. As a result, there is a concern that the imaging part will be falsely recognized. In addition, in a case in which auxiliary data used for diagnosis is provided on the basis of the false part recognition, there is a concern that diagnosis will be hindered.

The invention has been made in order to solve the problems of the related art and an object of the invention is to provide an ultrasound diagnostic apparatus and a method for controlling the ultrasound diagnostic apparatus that can prevent an error in part recognition.

According to the invention, there is provided an ultrasound diagnostic apparatus according to claim 1 of the appended claims.

In a case in which only one of the calculated degrees of confidence is greater than a predetermined threshold value, the dialogue display control unit may not display the dialogue on the display unit. In cases other than the case in which only one of the calculated degrees of confidence is greater than the predetermined threshold value, the dialogue display control unit may display the dialogue on the display unit.

In a case in which all of the calculated degrees of confidence are not greater than the predetermined threshold value, the dialogue display control unit may display the dialogue on the display unit. Alternatively, in a case in which a plurality of degrees of confidence among the calculated degrees of confidence are greater than the predetermined threshold value, the dialogue display control unit may display the dialogue on the display unit.

In a case in which the degree of confidence calculated for the collation pattern corresponding to an examination part that has been examined among the calculated degrees of confidence is greater than a predetermined threshold value, the dialogue display control unit displays the dialogue on the display unit.

The dialogue display control unit may display the dialogue formed according to the calculated degrees of confidence on the display unit.

The dialogue display control unit may display, on the display unit, the dialogue in which an examination part that corresponds to the collation pattern with a highest degree of confidence among the degrees of confidence calculated for each of the plurality of collation patterns is displayed so as to be larger than other examination parts.

The dialogue display control unit may display, on the display unit, the dialogue in which the examination parts corresponding to the plurality of collation patterns are displayed so as to be arranged in descending order of the degrees of confidence calculated for the plurality of collation patterns.

The dialogue display control unit may display, on the display unit, the dialogue in which the examination parts corresponding to the collation patterns, for which the degrees of confidence have been calculated, and values of the degrees of confidence are displayed.

The dialogue display control unit may display, on the display unit, the dialogue in which the examination part that has been examined is not displayed and an examination part that has not been examined is displayed.

The ultrasound diagnostic apparatus may further comprise a part confirmation unit that, in a case in which only one of the calculated degrees of confidence is greater than the predetermined threshold value, confirms that the examination part with the degree of confidence greater than the threshold value is the imaging part of the subject and confirms the imaging part of the subject on the basis of a check result of the user through the dialogue in cases other than the case in which only one of the calculated degrees of confidence is greater than the predetermined threshold value.

The ultrasound diagnostic apparatus may further comprise an imaging condition setting unit that sets imaging conditions corresponding to the imaging part confirmed by the part confirmation unit. The imaging unit may generate the B-mode image according to the imaging conditions set by the imaging condition setting unit.

According to the invention, there is provided a method for controlling an ultrasound diagnostic apparatus, according to claim 12 of the appended claims.

According to the disclosure, an ultrasound beam is transmitted to and received from a subject, using an ultrasound probe, and a received signal output from the ultrasound probe is converted into an image to generate an ultrasound image of the subject. A plurality of collation patterns that are stored in advance are read and the ultrasound image is collated using each of the plurality of read collation patterns to perform part recognition. The degrees of confidence for examination parts corresponding to each of the read collation patterns are calculated. It is determined whether to display, on a display unit, a dialogue for making a user check which of a plurality of examination parts, for which the degrees of confidence have been calculated, is an imaging part of the subject on the basis of the degrees of confidence. The dialogue is displayed on the display unit on the basis of the determination result. Therefore, it is possible to prevent an error in part recognition.

### BRIEF DESCRIPTION OF THE DRAWINGS

Fig. 1 is a block diagram illustrating the configuration of an ultrasound diagnostic apparatus according to Embodiment 1 of the disclosure.
Fig. 2 is a block diagram illustrating the internal configuration of a receiving unit.
Fig. 3 is a block diagram illustrating the internal configuration of a B-mode processing unit.
Fig. 4 is a flowchart illustrating the operation of Embodiment 1.
Fig. 5 is a flowchart illustrating a part recognition process according to Embodiment 1.
Fig. 6 is a diagram illustrating a dialogue displayed by the ultrasound diagnostic apparatus according to Embodiment 1.
Fig. 7 is a diagram illustrating a dialogue displayed by an ultrasound diagnostic apparatus according to Embodiment 2.
Fig. 8 is a diagram illustrating a dialogue displayed by a modification example of Embodiment 2.
Fig. 9 is a diagram illustrating a dialogue displayed by another modification example of Embodiment 2.
Fig. 10 is a diagram illustrating a dialogue displayed by still another modification example of Embodiment 2.
Fig. 11 is a diagram illustrating a dialogue displayed by yet another modification example of Embodiment 2.
Fig. 12 is a diagram illustrating a dialogue displayed by an ultrasound diagnostic apparatus according to Embodiment 3.
Fig. 13 is a diagram illustrating a dialogue displayed by a modification example of Embodiment 3.
Fig. 14 is a diagram illustrating a dialogue displayed by another modification example of Embodiment 3.
Fig. 15 is a diagram illustrating a dialogue displayed by still another modification example of Embodiment 3.

### DESCRIPTION OF THE PREFERRED EMBODIMENTS

Hereinafter, embodiments of the invention will be described with reference to the accompanying drawings.

### Embodiment 1

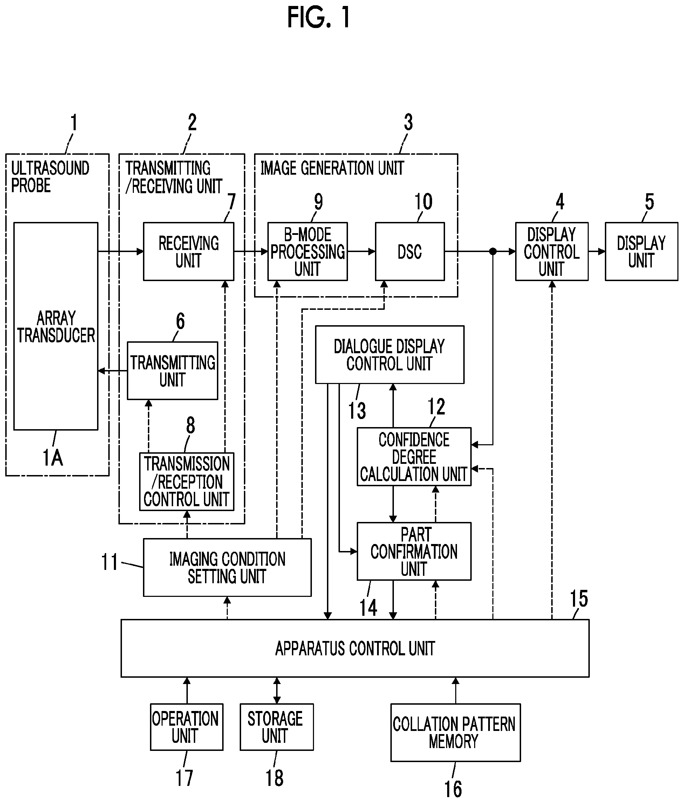

Fig. 1 illustrates the configuration of an ultrasound diagnostic apparatus according to Embodiment 1 of the invention. The ultrasound diagnostic apparatus includes an ultrasound probe 1 provided with an array transducer 1A. An image generation unit 3 is connected to the ultrasound probe 1 through a transmitting/receiving unit 2 and a display unit 5 is connected to the image generation unit 3 through a display control unit 4.

The transmitting/receiving unit 2 includes a transmitting unit 6 and a receiving unit 7 that are connected to the array transducer 1A of the ultrasound probe 1 and a transmission/reception control unit 8 that is connected to the transmitting unit 6 and the receiving unit 7. The image generation unit 3 includes a brightness-mode (B-mode) processing unit 9 that is connected to the receiving unit 7 of the transmitting/receiving unit 2 and a digital scan converter (DSC) 10 that is connected to the B-mode processing unit 9. The display control unit 4 is connected to the DSC 10. An imaging condition setting unit 11 is connected to the transmission/reception control unit 8 of the transmitting/receiving unit 2 and the B-mode processing unit 9 and the DSC 10 of the image generation unit 3.

In addition, a confidence degree calculation unit 12 is connected to the DSC 10. A dialogue display control unit 13 and a part confirmation unit 14 are connected to the confidence degree calculation unit 12. The dialogue display control unit 13 is connected to the part confirmation unit 14.

An apparatus control unit 15 is connected to the imaging condition setting unit 11, the confidence degree calculation unit 12, the dialogue display control unit 13, and the part confirmation unit 14. In addition, a collation pattern memory 16, an operation unit 17, and a storage unit 18 are connected to the apparatus control unit 15.

The array transducer 1A of the ultrasound probe 1 includes a plurality of ultrasound transducers that are one-dimensionally or two-dimensionally arranged. Each of the ultrasound transducers transmits ultrasonic waves in response to a driving signal supplied from the transmitting unit 6. In addition, each of the ultrasound transducers receives ultrasound echoes from a subject and generates a received signal. Each ultrasound transducer is, for example, a transducer in which electrodes are formed at both ends of a piezoelectric body made of piezoelectric ceramic typified by lead zirconate titanate (PZT), a polymer piezoelectric element typified by polyvinylidene difluoride (PVDF), or a piezoelectric crystal typified by lead magnesium niobate-lead titanate (PMN-PT).

In a case in which a pulsed voltage or a continuous-wave voltage is applied to the electrodes of the transducer, the piezoelectric body is expanded and contracted and pulsed or continuous ultrasonic waves are generated from each transducer. The ultrasonic waves are combined to form an ultrasound beam. In addition, each transducer receives propagated ultrasonic waves, is expanded and contracted, and generates an electric signal. The electric signal is output as a received ultrasound signal.

The transmitting/receiving unit 2 transmits and receives an ultrasound beam according to the set ultrasound beam scanning conditions and the image generation unit 3 generates a B-mode image signal according to the set ultrasound image generation conditions. Therefore, the transmitting/receiving unit 2 and the image generation unit 3 form an imaging unit. In addition, imaging conditions for the imaging unit are formed by ultrasound beam scanning conditions for the transmitting/receiving unit 2 and ultrasound image generation conditions for the image generation unit 3.

The transmitting unit 6 of the transmitting/receiving unit 2 includes, for example, a plurality of pulse generators, adjusts the amount of delay of each driving signal such that the ultrasonic waves transmitted from a plurality of ultrasound transducers in the array transducer 1A form an ultrasound beam, on the basis of a transmission delay pattern selected according to a control signal from the transmission/reception control unit 8, and supplies the driving signals to the plurality of ultrasound transducers.

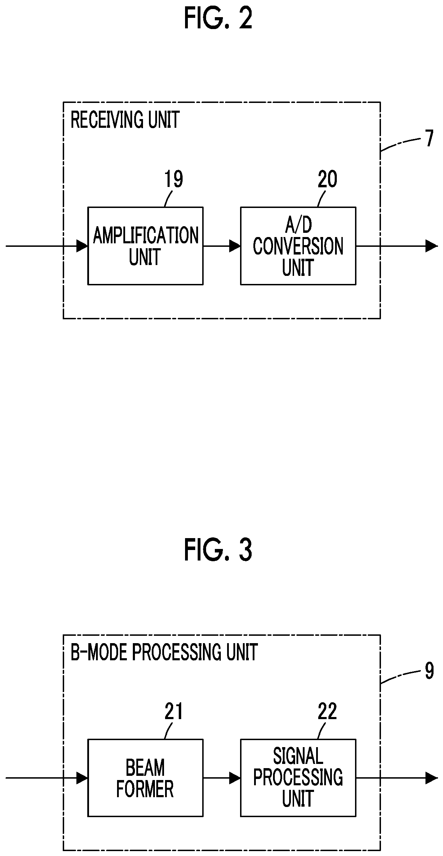

As illustrated in Fig. 2, the receiving unit 7 has a configuration in which an amplification unit 19 and an analog/digital (A/D) conversion unit 20 are sequentially connected in series. The receiving unit 7 amplifies the received signals transmitted from each ultrasound transducer of the array transducer 1A with the amplification unit 19 and performs A/D conversion for the received signals with the A/D conversion unit 20 to generate digital received data.

The transmission/reception control unit 8 controls the transmitting unit 6 and the receiving unit 7 on the basis of various control signals transmitted from the apparatus control unit 15 such that the transmission of an ultrasound pulse to a subject and the reception of an ultrasound echo from the subject are repeated at a pulse repetition frequency (PRF) interval.

The B-mode processing unit 9 of the image generation unit 3 has a configuration in which a beam former 21 and a signal processing unit 22 are sequentially connected in series, as illustrated in Fig. 3. The beam former 21 applies a delay to each received data item output from the receiving unit 7 of the transmitting/receiving unit 2 according to a sound speed or a sound speed distribution set on the basis of a reception delay pattern selected according to a control signal from the imaging condition setting unit 11 and adds the received data to perform a reception focusing process. A sound ray signal in which the focus of an ultrasound echo subjected to phasing addition is narrowed is generated by the reception focusing process.

The signal processing unit 22 corrects the attenuation of the sound ray signal generated by the beam former 21 depending on a distance according to the depth of the reflection position of ultrasonic waves and then performs an envelope detection process. In addition, the signal processing unit 22 performs various types of necessary image processing including a gradation process to generate a B-mode image signal which is tomographic image information related to the tissues of the subject.

The DSC 10 of the image generation unit 3 converts the B-mode image signal generated by the signal processing unit 22 into an image signal based on a general television signal scanning system (raster conversion).

The display control unit 4 displays a B-mode image on the display unit 5 on the basis of the B-mode image signal generated by the image generation unit 3.

The display unit 5 includes a display device, such as a liquid crystal display (LCD), and displays the B-mode image under the control of the display control unit 4.

The operation unit 17 is used by a user to perform an input operation and may include, for example, a keyboard, a mouse, a trackball, and a touch panel.

A plurality of collation patterns corresponding to a plurality of examination parts of the subject are stored in the collation pattern memory 16 in advance.

The confidence degree calculation unit 12 reads a plurality of collation patterns from the collation pattern memory 16, compares each of the read collation patterns with the B-mode image signal generated by the image generation unit 3, and calculates the degree of confidence for each of the read collation patterns. Here, the degree of confidence means the degree of similarity between the B-mode image signal and the collation pattern. That is, the degree of confidence is an index indicating the degree of confidence that an imaging part of the subject will be an examination part corresponding to the collation pattern.

The dialogue display control unit 13 determines whether to display, on the display unit 5, a dialogue for making the user check which of the examination parts corresponding to the collation patterns, for which the degrees of confidence have been calculated, is the imaging part of the subject, on the basis of the degrees of confidence calculated by the confidence degree calculation unit 12. Specifically, in a case in which only one of the calculated degrees of confidence is greater than a predetermined threshold value, the imaging part of the subject can be confirmed to be an examination part corresponding to the collation pattern with the calculated degree of confidence. Therefore, the dialogue is determined not to be displayed on the display unit 5.

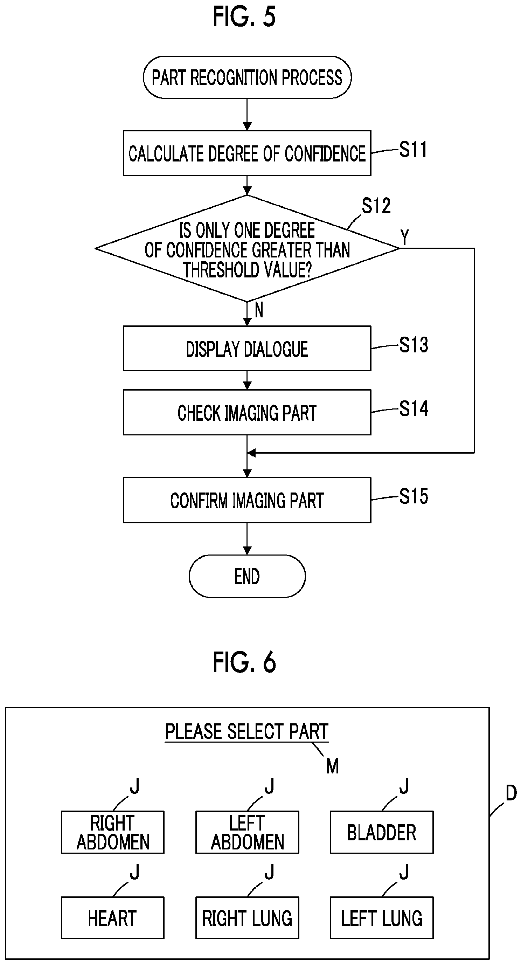

On the other hand, in cases other than the case in which only one of the calculated degrees of confidence is greater than the predetermined threshold value, it is difficult to confirm which of the examination parts is the imaging part of the subject. Therefore, for example, a dialogue formed according to a plurality of calculated degrees of confidence is determined to be displayed on the display unit 5 as illustrated in Fig. 6. Specifically, a case in which all of the calculated degrees of confidence are not greater than the predetermined threshold value or a case in which the plurality of calculated degrees of confidence are greater than the predetermined threshold value is given as an example.

In a case in which the degree of confidence calculated for the collation pattern corresponding to the examination part which has been examined is greater than the predetermined threshold value, there is a concern that the same examination part will be repeatedly examined and it is necessary to prompt the user to make a check. Therefore, for example, the dialogue illustrated in Fig. 6 is determined to be displayed on the display unit 5.

In addition, the dialogue display control unit 13 outputs the determination result to the apparatus control unit 15 and the part confirmation unit 14.

In a case in which the determination result indicating that the dialogue is displayed on the display unit 5 is output, information for forming the dialogue is output to the apparatus control unit 15 together with the determination result. On the other hand, in a case in which the determination result indicating that the dialogue is not displayed on the display unit 5 is output, information indicating the examination part corresponding to the collation pattern with the calculated degree of confidence greater than the predetermined threshold value is output to the part confirmation unit 14 together with the determination result.

The apparatus control unit 15 controls the display control unit 4 such that the dialogue is displayed on the display unit 5, on the basis of the determination result indicating that the dialogue is displayed on the display unit 5 from the dialogue display control unit 13.

In addition, in a case in which the check result of the user based on the dialogue displayed on the display unit 5 is input through the operation unit 17, the apparatus control unit 15 outputs the check result to the part confirmation unit 14.

Furthermore, the apparatus control unit 15 controls the display control unit 4, the imaging condition setting unit 11, the confidence degree calculation unit 12, and the part confirmation unit 14 on the basis of commands input by the user through the operation unit 17.

In a case in which the determination result indicating that the dialogue is displayed on the display unit 5 is input, the part confirmation unit 14 confirms the imaging part of the subject on the basis of the check result of the imaging part of the subject by the user from the apparatus control unit 15. On the other hand, in a case in which the determination result indicating that the dialogue is not displayed on the display unit 5 is input, the part confirmation unit 14 confirms that the imaging part of the subject is the examination part indicated by the information which has been input together with the determination result.

The imaging condition setting unit 11 stores imaging conditions corresponding to a plurality of examination parts in advance and sets the imaging conditions corresponding to the examination part confirmed by the part confirmation unit 14.

The storage unit 18 stores, for example, an operation program. For example, a recording medium, such as a hard disk, a flexible disk, a magneto-optical disk (MO), a magnetic tape (MT), a random access memory (RAM), a compact disk read only memory (CD-ROM), a digital versatile disk read only memory (DVD-ROM), a secure digital card (SD card), a compact flash card (CF card), or a universal serial bus memory (USB memory), or a server may be used as the storage unit 18.

The image generation unit 3, the display control unit 4, the transmission/reception control unit 8 of the transmitting/receiving unit 2, the imaging condition setting unit 11, the confidence degree calculation unit 12, the dialogue display control unit 13, the part confirmation unit 14, and the apparatus control unit 15 are formed by a central processing unit (CPU) and an operation program that causes the CPU to perform various processes. However, these units may be formed by digital circuits. In addition, some or all of the image generation unit 3, the display control unit 4, the transmission/reception control unit 8 of the transmitting/receiving unit 2, the imaging condition setting unit 11, the confidence degree calculation unit 12, the dialogue display control unit 13, the part confirmation unit 14, and the apparatus control unit 15 may be integrated into one CPU.

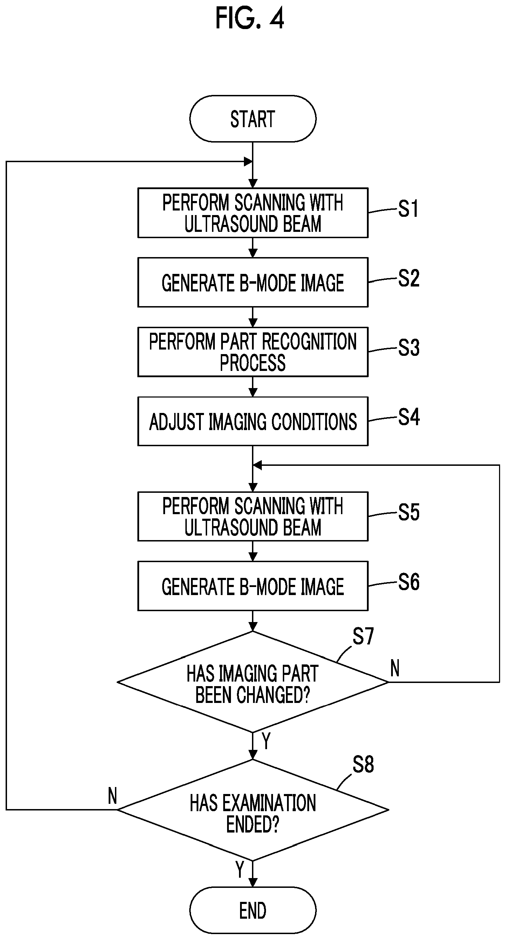

Next, the operation of Embodiment 1 will be described with reference to a flowchart illustrated in Fig. 4.

First, in Step S1, the transmitting/receiving unit 2 performs the transmission and reception of an ultrasound beam and scanning, using the plurality of ultrasound transducers in the array transducer 1A of the ultrasound probe 1. A received signal from each ultrasound transducer that has received ultrasound echoes from the subject is output to the receiving unit 7. The receiving unit 7 performs amplification and A/D conversion for the received signal to generate received data.

Then, in Step S2, the received data is input to the image generation unit 3. The B-mode processing unit 9 performs a reception focusing process for the received data and the DSC 10 converts received data to generate a B-mode image signal. The B-mode image signal is output from the image generation unit 3 to the display control unit 4. The B-mode image is displayed on the display unit 5.

The B-mode image signal generated by the image generation unit 3 is also output to the confidence degree calculation unit 12. Then, in Step S3, the confidence degree calculation unit 12 collates the B-mode image signal to perform a part recognition process. Specifically, the process in Steps S11 to S15 in the flowchart illustrated in Fig. 5 is performed.

In Step S11, first, the confidence degree calculation unit 12 reads a plurality of collation patterns corresponding to a plurality of examination parts from the collation pattern memory 16 and collates the B-mode image signal with each of the read collation patterns to perform part recognition.

For example, the confidence degree calculation unit 12 reads a plurality of collation patterns corresponding to the right abdomen, the left abdomen, the bladder, the heart, the right lung, and the left lung from the collation pattern memory 16. The confidence degree calculation unit 12 compares the B-mode image signal with the collation pattern corresponding to the right abdomen and calculates the degree of similarity between the B-mode image signal and the collation pattern corresponding to the right abdomen, that is, the degree of confidence that the imaging part of the subject will be the right abdomen.

Similarly, the confidence degree calculation unit 12 compares the B-mode image signal with each of the plurality of collation patterns corresponding to the left abdomen, the bladder, the heart, the right lung, and the left lung and calculates the degree of confidence for each of the collation patterns. The calculated degrees of confidence are output to the dialogue display control unit 13.

A known matching technique can be used to compare the B-mode image signal with the read collation patterns and to calculate the degree of confidence. For example, the degree of confidence can be calculated by a machine learning method, a template matching method, or a general image recognition method.

The dialogue display control unit 13 stores a predetermined threshold value that has been set for the degree of confidence in advance. In Step S12, the dialogue display control unit 13 determines whether only one of the degrees of confidence calculated by the confidence degree calculation unit 12 is greater than the threshold value. In a case in which the degree of confidence is less than the threshold value, it is difficult to have confidence that the imaging part of the subject is the examination part corresponding to the collation pattern for which the degree of confidence has been calculated.

In a case in which only one of the calculated degrees of confidence is greater than the threshold value, the dialogue display control unit 13 determines not to display the dialogue on the display unit 5. In cases other than the case in which only one of the calculated degrees of confidence is greater than the threshold value, the dialogue display control unit 13 determines to display the dialogue on the display unit 5.

For example, as the case in which only one of the calculated degrees of confidence is greater than the threshold value, the following case is considered: a case in which it is determined that, among the degrees of confidence calculated for the collation patterns corresponding to the right abdomen, the left abdomen, the bladder, the heart, the right lung, and the left lung, only the degree of confidence calculated for the collation pattern corresponding to the right abdomen is greater than the predetermined threshold value. In this case, the dialogue display control unit 13 outputs the determination result indicating that the dialogue is not displayed on the display unit 5 to the apparatus control unit 15 and the part confirmation unit 14. In addition to the determination result, information indicating that the right abdomen corresponds to the collation pattern with the calculated degree of confidence greater than the predetermined threshold value is output to the part confirmation unit 14. Then, Steps S13 and S 14 are omitted and the process proceeds to Step S 15.

In contrast, as a case other than the case in which only one of the calculated degrees of confidence is greater than the threshold value, the following case is considered: a case in which it is determined that all of the degrees of confidence calculated for the collation patterns corresponding to the right abdomen, the left abdomen, the bladder, the heart, the right lung, and the left lung are less than the predetermined threshold value. In this case, the dialogue display control unit 13 determines to display the dialogue on the display unit 5 in order to make the user check which of the right abdomen, the left abdomen, the bladder, the heart, the right lung, and the left lung is the imaging part of the subject.

In addition, as the case other than the case in which only one of the calculated degrees of confidence is greater than the threshold value, the following case is considered: a case in which it is determined that the degrees of confidence calculated for the collation patterns corresponding to the right abdomen and the heart are greater than the predetermined threshold value. In this case, the dialogue display control unit 13 determines to display the dialogue with the configuration illustrated in Fig. 6 on the display unit 5 in order to make the user check which of the right abdomen, the left abdomen, the bladder, the heart, the right lung, and the left lung is the examination part.

For example, in a case in which the right abdomen has been examined and the degree of confidence calculated for the collation pattern corresponding to the right abdomen is determined to be greater than the predetermined threshold value, there is a concern that the right abdomen will be repeatedly examined. In this case, the dialogue display control unit 13 determines to display the dialogue with the configuration illustrated in Fig. 6 on the display unit 5 in order to make the user check which of the right abdomen, the left abdomen, the bladder, the heart, the right lung, and the left lung is the examination part.

Then, the dialogue display control unit 13 outputs the determination result indicating that the dialogue is displayed on the display unit 5 to the apparatus control unit 15 and the part confirmation unit 14. In addition, the dialogue display control unit 13 outputs information for forming the dialogue to the apparatus control unit 15 together with the determination result.

In Step S 13, the apparatus control unit 15 controls the display control unit 4 on the basis of the information for forming the dialogue from the dialogue display control unit 13 such that a dialogue D illustrated in Fig. 6 is displayed on the display unit 5.

The dialogue D includes a plurality of icons J indicating the names of the examination parts corresponding to each of the collation patterns, for which the degrees of confidence have been calculated, and a message M that prompts the user to select the examination part. The dialogue D illustrated in Fig. 6 is used to make the user check which of the right abdomen, the left abdomen, the bladder, the heart, the right lung, and the left lung is the imaging part of the subject.

In Step S14, the user operates the operation unit 17 to select any one of the plurality of icons J displayed in the dialogue D and checks which of the right abdomen, the left abdomen, the bladder, the heart, the right lung, and the left lung is the imaging part of the subject.

For example, in a case in which the user selects the icon J indicating the right abdomen, the check result indicating that the imaging part of the subject is the right abdomen is input to the apparatus control unit 15 through the operation unit 17. The check result is output from the apparatus control unit 15 to the part confirmation unit 14.

Then, in Step S15, the part confirmation unit 14 confirms that the imaging part of the subject is the right abdomen on the basis of the check result indicating that the imaging part of the subject is the right abdomen which has been output from the apparatus control unit 15. As such, even in a case in which all of the degrees of confidence calculated for the collation patterns corresponding to the right abdomen, the left abdomen, the bladder, the heart, the right lung, and the left lung are less than the predetermined threshold value, the dialogue D is displayed on the display unit 5 such that the user checks which of the examination parts is the imaging part of the subject. Therefore, it is possible to prevent an error in part recognition.

As described above, even in a case in which the degrees of confidence calculated for the collation patterns corresponding to two parts, that is, the right abdomen and the heart are greater than the predetermined threshold value, the dialogue D illustrated in Fig. 6 is displayed on the display unit 5. Then, the user checks which of the right abdomen, the left abdomen, the bladder, the heart, the right lung, and the left lung is the examination part.

As such, in a case in which a plurality of calculated degrees of confidence are greater than the predetermined threshold value, the dialogue D is displayed on the display unit 5 such that the user checks the imaging part. Therefore, it is possible to prevent an error in part recognition.

As described above, even in a case in which the right abdomen has been examined and the degree of confidence calculated for the collation pattern corresponding to the right abdomen is determined to be greater than the predetermined threshold value, the dialogue D illustrated in Fig. 6 is displayed on the display unit 5. Then, the user checks which of the right abdomen, the left abdomen, the bladder, the heart, the right lung, and the left lung is the examination part.

With this configuration, it is possible to prevent the user from repeatedly examining the examination part that has been examined.

In a case in which the dialogue display control unit 13 outputs the determination result indicating that the dialogue D is not displayed on the display unit 5 to the apparatus control unit 15 and the part confirmation unit 14 in Step S12 as described above, the dialogue display control unit 13 outputs, to the part confirmation unit 14, information indicating that the right abdomen corresponds to the collation pattern with the calculated degree of confidence greater than the predetermined threshold value together with the determination result. Then, Steps S13 and S14 are omitted and the part confirmation unit 14 confirms that the imaging part of the subject is the right abdomen in Step S15. In addition, the dialogue D is not displayed on the display unit 5 and the check of the examination part by the user is omitted, according to the determination result input from the dialogue display control unit 13 to the apparatus control unit 15.

In a case in which the part confirmation unit 14 outputs, for example, the confirmation result indicating that the imaging part is the right abdomen, the imaging condition setting unit 11 adjusts the imaging conditions in Step S4 of the flowchart illustrated in Fig. 4. The imaging condition setting unit 11 stores a plurality of imaging conditions in advance, selects imaging conditions on the basis of the output confirmation result, and controls the transmitting/receiving unit 2 and the image generation unit 3 such that imaging is performed for the subsequent frames under the selected imaging conditions.

Then, in Step S5, the transmitting/receiving unit 2 is controlled according to ultrasound beam scanning conditions included in the imaging conditions selected by the imaging condition setting unit 11. Then, in Step S6, the image generation unit 3 is controlled according to ultrasound image generation conditions included in the imaging conditions selected by the imaging condition setting unit 11. In addition, the B-mode image signal is output from the image generation unit 3 to the display control unit 4. In this way, the B-mode image suitable for diagnosing the right abdomen can be displayed on the display unit 5.

Then, in Step S7, the confidence degree calculation unit 12 determines whether the imaging part of the B-mode image signal has been changed. For example, in a case in which the examination part is changed from the right abdomen to the left abdomen and the imaging part is changed, the confidence degree calculation unit 12 determines that the imaging part has been changed. Specifically, in general, in a case in which the imaging part is changed, the probe is separated from the surface of the body and emits ultrasonic waves to the air. Therefore, it is possible to determine whether the imaging part has been changed by detecting the aerial emission state (a state in which a reflected signal is not obtained).

Then, Steps S5 to S7 are repeated until the confidence degree calculation unit 12 determines that the imaging part has been changed in Step S7 and the right abdomen which is the imaging part is continuously diagnosed.

Then, for example, in a case in which it is determined in Step S7 that the imaging part has been changed the right abdomen to the left abdomen, it is determined whether examination has ended in Step S8. Then, in a case in which it is determined that examination is continuously performed, the process returns to Step S 1 and a B-mode image signal including the left abdomen is generated through Step S 1 and S2. Then, in Step S3, a part recognition process is performed. As such, Steps S 1 to S8 are repeated until examination is determined to end in Step S8.

For the plurality of read collation patterns, the degree of confidence is calculated for each frame. The degrees of confidence corresponding to a predetermined number of frames are calculated. In a case in which all of the degrees of confidence calculated for all of the frames are less than the predetermined threshold value, the dialogue D may be displayed on the display unit 5. As such, since the degrees of confidence corresponding to a plurality of frames are calculated to recognize the imaging part, it is possible to effectively prevent an error in part recognition.

Even in a case in which the degree of confidence calculated for the collation pattern corresponding to the examination part which has been examined is greater than the predetermined threshold value, the dialogue D may be displayed on the display unit 5. In this case, it is possible to prevent the imaging part of the subject from being falsely recognized as the examination part that has been examined.

### Embodiment 2

In Embodiment 1, the dialogue D illustrated in Fig. 6 is displayed on the display unit 5. However, in Embodiment 2, a dialogue D with a configuration different from that of the dialogue D illustrated in Fig. 6 is displayed on the display unit 5.

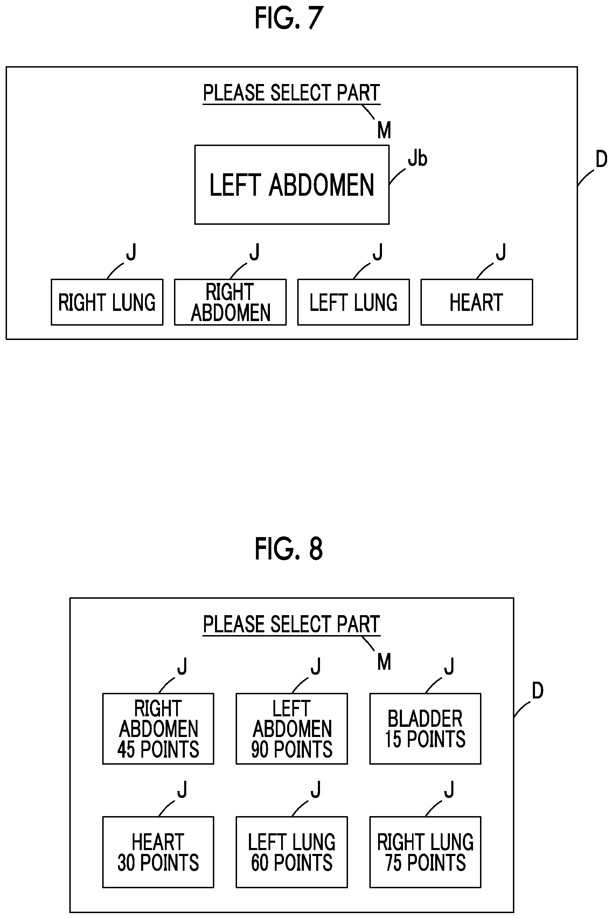

For example, the confidence degree calculation unit 12 reads a plurality of collation patterns corresponding to the right abdomen, the left abdomen, the bladder, the heart, the right lung, and the left lung from the collation pattern memory 16 and calculates the degrees of confidence for each of the collation patterns. It is assumed that the left abdomen has the highest degree of confidence, followed by the right lung, the left lung, the right abdomen, the heart, and the bladder. Then, in a case in which all of the degrees of confidence are less than a predetermined threshold value, a dialogue D illustrated in Fig. 7 is displayed on the display unit 5.

An icon Jb indicating the left abdomen corresponding to the collation pattern with the highest degree of confidence among the calculated degrees of confidence is displayed so as to be larger than a plurality of icons J indicating the other examination parts, that is, the lung, the right abdomen, and the heart. Therefore, the user easily checks that the imaging part of the subject is the left abdomen corresponding to the collation pattern with the highest degree of confidence.

In addition, an icon indicating the bladder corresponding to the collation pattern with the lowest degree of confidence among the calculated degrees of confidence is not displayed in the dialogue D illustrated in Fig. 7. Therefore, it is possible to prevent the user from falsely recognizing that the imaging part of the subject is the bladder corresponding to the collation pattern with the lowest degree of confidence.

As in a dialogue D illustrated in Fig. 8, a plurality of icons J in which the examination parts corresponding to the collation patterns, for which the degrees of confidence have been calculated, and the values of the calculated degrees of confidence are displayed may be displayed. The user can check that the imaging part of the subject is the left abdomen corresponding to the icon J indicating the maximum value from the values of the calculated degrees of confidence displayed in the plurality of icons J.

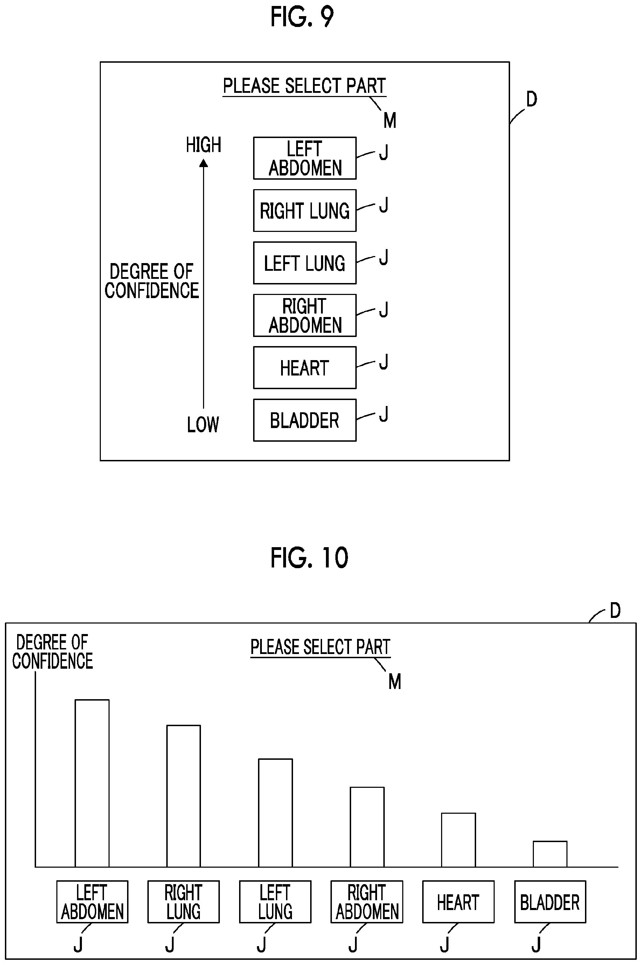

As in a dialogue D illustrated in Fig. 9, a plurality of icons J indicating the examination parts corresponding to the collation patterns may be displayed so as to be arranged in descending order of the calculated degrees of confidence. In the dialogue D illustrated in Fig. 9, a plurality of icons J indicating the examination parts are displayed so as to be arranged in the order of the left abdomen, the right lung, the left lung, the right abdomen, the heart, and the bladder from the position close to the message M. Therefore, the user can recognize the order of the calculated degrees of confidence.

As in a dialogue D illustrated in Fig. 10, a graph in which the vertical axis indicates the degree of confidence and the horizontal axis indicates a plurality of icons J indicating the examination parts corresponding to the collation patterns, for which the degrees of confidence have been calculated, may be displayed. The graph enables the user to recognize the order of the calculated degrees of confidence.

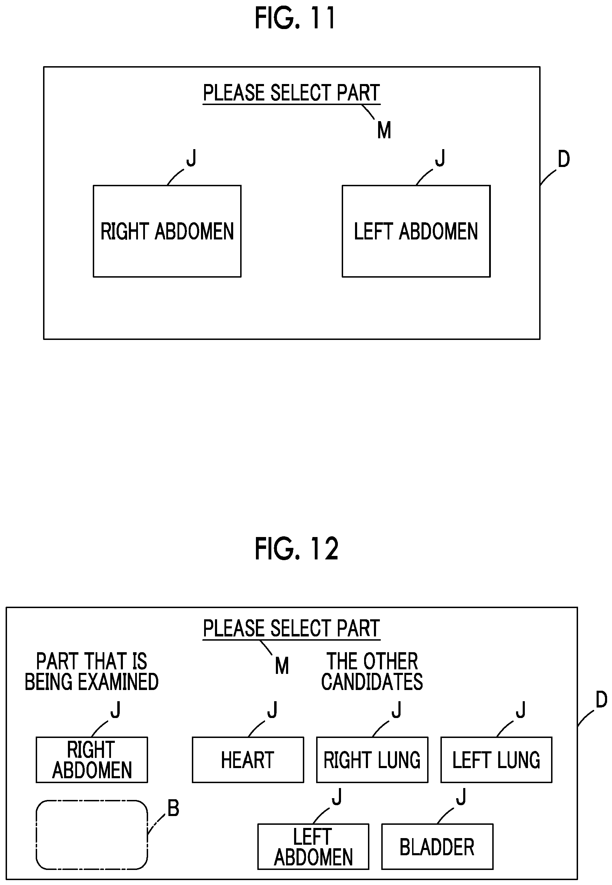

In a case in which the plurality of calculated degrees of confidence are greater than the predetermined threshold value, only the icon J that corresponds to the examination part corresponding to the collation pattern with the calculated degree of confidence greater than the threshold value may be displayed in the dialogue D. For example, in a case in which the degrees of confidence calculated for the collation patterns corresponding to the right abdomen and the left abdomen are greater than the predetermined threshold value, only the icons J corresponding to the right abdomen and the left abdomen may be displayed as in a dialogue D illustrated in Fig. 11. In this case, the icons which correspond to the bladder, the heart, the right lung, and the left lung corresponding to the collation patterns with the calculated degrees of confidence less than the predetermined threshold value are not displayed in the dialogue D. Therefore, it is possible to prevent the user from falsely recognizing that the imaging part of the subject is any one of the bladder, the heart, the right lung, and the left lung.

In the dialogues D illustrated in Figs. 8 to 11, the icon indicating the left abdomen corresponding to the collation pattern with the highest degree of confidence may be displayed as the icon Jb that is displayed so as to be larger than the other icons J.

As such, in Embodiment 2, the dialogue D is formed on the basis of the calculated degree of confidence. Therefore, the user easily checks the imaging part of the subject and it is possible to effectively prevent an error in part recognition.

### Embodiment 3

In Embodiment 3, a plurality of predetermined examination parts are continuously examined. Therefore, a plurality of collation patterns corresponding to the plurality of predetermined examination parts are read from the collation pattern memory 16 and the degree of confidence is calculated for each of the read collation patterns.

For example, in a case in which the right abdomen, the left abdomen, the bladder, the heart, the right lung, and the left lung are examined, the confidence degree calculation unit 12 reads the collation patterns corresponding to the right abdomen, the left abdomen, the bladder, the heart, the right lung, and the left lung from the collation pattern memory 16 and calculates the degree of confidence for each of the read collation patterns.

In a case in which all of the calculated degrees of confidence are less than the predetermined threshold value, a dialogue D illustrated in Fig. 12 is displayed on the display unit 5. In the dialogue D illustrated in Fig. 12, an icon J indicating the right abdomen as "a part that is being examined" and a plurality of icons J indicating the left abdomen, the bladder, the heart, the right lung and the left lung as "the other candidates" are displayed. The term "the part that is being examined" means an examination part as the current examination target and the term "the other candidates" means the examination parts other than "the part that is being examined".

As such, in a case in which a plurality of predetermined examination parts are continuously examined and all of the calculated degrees of confidence are less than the predetermined threshold value, the dialogue D illustrated in Fig. 12 enables the user to recognize the examination part as the current examination target and the other examination parts and to check the imaging part. Therefore, it is possible to prevent an error in part recognition.

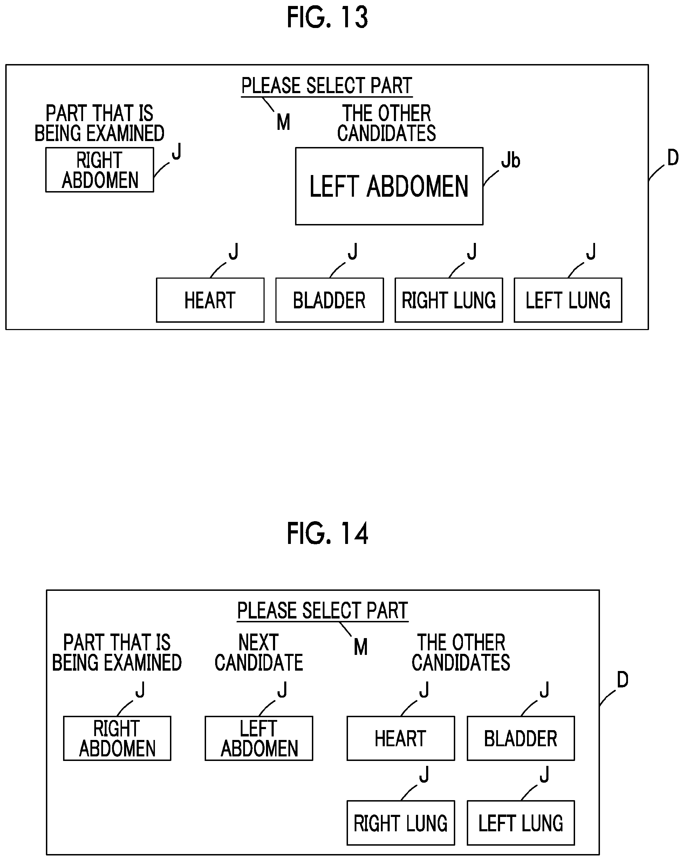

An icon indicating the examination part corresponding to the collation pattern with the highest degree of confidence among the calculated degrees of confidence may be displayed so as to be larger than icons indicating the other examination parts. For example, in a case in which the degree of confidence calculated for the collation pattern corresponding to the left abdomen is the highest among the calculated degrees of confidence, an icon Jb indicating the left abdomen is displayed so as to be larger than a plurality of icons J indicating the right abdomen, the heart, the bladder, the right lung, and the left lung as in a dialogue D illustrated in Fig. 13.

As such, in a case in which the icon Jb indicating the left abdomen displayed as one of "the other candidates" is displayed so as to be larger than the icon J indicating the right abdomen as the current examination target, it is possible to effectively prevent an error in part recognition even though the user examines the left abdomen which is not the current examination target.

In a case in which an examination order is predetermined and an extended focused assessment with sonography for trauma (eFAST) examination that continuously examines, for example, the right abdomen, the left abdomen, the bladder, the heart, the right lung, and the left lung in this order is performed, the schema image illustrated in Fig. 6 in JP2015-29619A may be added to a blank B in the dialogue D illustrated in Fig. 12. The addition of the schema image makes it easy for the user to recognize the progress of the eFAST examination.

As in a dialogue D illustrated in Fig. 14, in a case in which "the part that is being examined" is the right abdomen in the eFAST examination, the left abdomen which is the next examination target may be displayed as "the next candidate".

As such, the examination part which is the next examination target is displayed as "the next candidate" according to a predetermined order in the eFAST examination. Therefore, the user can easily check the imaging part of the subject.

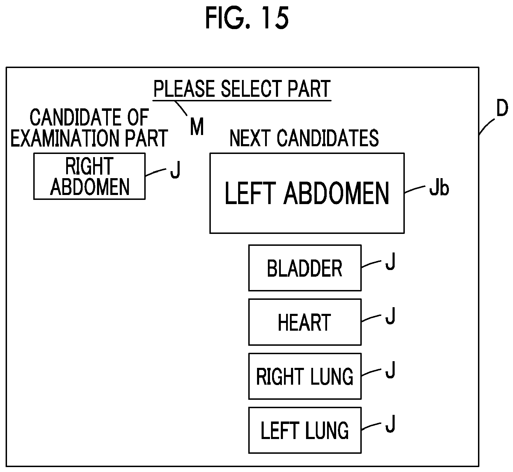

As in a dialogue D illustrated in Fig. 15, an examination part as the current examination target and a plurality of examination parts as the next examination targets may be displayed so as to be arranged in the order of examination. For example, in a case in which the examination part as the current examination target is the right abdomen, the icons J and Jb are displayed in the order of the left abdomen as the next examination target and the bladder, the heart, the right lung, and the left lung which are to be examined after the left abdomen. In a case in which the degree of confidence calculated for the collation pattern corresponding to the left abdomen is the highest among the calculated degrees of confidence, the icon Jb indicating the left abdomen is displayed so as to be larger than a plurality of icons J indicating the right abdomen, the bladder, the heart, the right lung, and the left lung.

As such, the user can recognize the order in which the examination parts are examined and the examination part corresponding to the collation pattern with the highest degree of confidence. Therefore, it is possible to effectively prevent an error in part recognition.

In addition, since the examination part that has been examined is not displayed as the icon J in the dialogue D, it is possible to prevent the user from checking the examination part that has been examined as the imaging part of the subject. In addition, with the progress of examination, the number of icons J indicating the unexamined parts displayed in the dialogue D is reduced. Therefore, the user can recognize the progress of examination.

In addition, a second threshold value that is less than the predetermined threshold value is set in advance. In a case in which the calculated degree of confidence is less than the second threshold value, the degree of confidence may not be calculated for the other read collation patterns and the dialogue D may be displayed on the display unit 5.

For example, in the eFAST examination, in a case in which the degree of confidence calculated for the collation pattern corresponding to the right abdomen as the current examination target is less than the second threshold value, the possibility that an examination part different from the right abdomen will be examined is high. In this case, the calculation of the degree of confidence for the collation patterns corresponding to the left abdomen, the bladder, the heart, the right lung, and the left lung as the next examination targets is omitted and the dialogue D is displayed on the display unit 5. Therefore, it is possible to rapidly prompt the user to check the examination part.

### Explanation of References

1: ultrasound probe
1A: array transducer
2: transmitting/receiving unit
3: image generation unit
4: display control unit
5: display unit
6: transmitting unit
7: receiving unit
8: transmission/reception control unit
9: B-mode processing unit
10: DSC
11: imaging condition setting unit
12: confidence degree calculation unit
13: dialogue display control unit
14: part confirmation unit
15: apparatus control unit
16: collation pattern memory
17: information input unit
18: storage unit
19: amplification unit
20: A/D conversion unit
21: beam former
22: signal processing unit
D: dialogue
J: icon
Jb: icon larger than other icons
M: message
B: blank

## Claims

1. An ultrasound diagnostic apparatus comprising:
an ultrasound probe (1);
an imaging unit (3) configured to transmit an ultrasound beam to and receive an ultrasound echo from an imaging part of a subj ect using the ultrasound probe and to convert a received signal output from the ultrasound probe into an image to generate a B-mode image of the subject;
a display unit (5);
a collation pattern memory (16) configured to store a plurality of collation patterns corresponding to a plurality of examination parts of the subject in advance;
a confidence degree calculation unit (12) configured to read the plurality of collation patterns from the collation pattern memory, to collate the B-mode image generated by the imaging unit (3) with each of the plurality of read collation patterns to perform part recognition, and to calculate degrees of confidence for the examination parts corresponding to each of the read collation patterns; and
a dialogue display control unit (13) configured to determine whether to display, on the display unit (5), a dialogue (D) for making a user check which of the plurality of examination parts, for which the degrees of confidence have been calculated, is the imaging part of the subject on the basis of the degrees of confidence and to display the dialogue on the display unit on the basis of a determination result;
**characterized in that**, in a case in which the degree of confidence calculated for the collation pattern corresponding to an examination part that has been examined among the calculated degrees of confidence is greater than a predetermined threshold value, the dialogue display control unit (13) is configured to display the dialogue (D) on the display unit (5).

2. The ultrasound diagnostic apparatus according to claim 1,
wherein, in a case in which only one of the calculated degrees of confidence is greater than a predetermined threshold value, the dialogue display control unit (13) is configured to not display the dialogue (D) on the display unit (5), and
in cases other than the case in which only one of the calculated degrees of confidence is greater than the predetermined threshold value, the dialogue display control unit is configured to display the dialogue on the display unit.

3. The ultrasound diagnostic apparatus according to claim 2,
wherein, in a case in which all of the calculated degrees of confidence are not greater than the predetermined threshold value, the dialogue display control unit (13) is configured to display the dialogue (D) on the display unit (5).

4. The ultrasound diagnostic apparatus according to claim 2,
wherein, in a case in which a plurality of degrees of confidence among the calculated degrees of confidence are greater than the predetermined threshold value, the dialogue display control unit (13) is configured to display the dialogue (D) on the display unit (5).

5. The ultrasound diagnostic apparatus according to any one of claims 1 to 4,
wherein the dialogue display control unit (13) is configured to display the dialogue (D) formed according to the calculated degrees of confidence on the display unit (5).

6. The ultrasound diagnostic apparatus according to claim 5,
wherein the dialogue display control unit (13) is configured to display, on the display unit (5), the dialogue (D) in which an examination part that corresponds to the collation pattern with a highest degree of confidence among the degrees of confidence calculated for each of the plurality of collation patterns is displayed so as to be larger than other examination parts.

7. The ultrasound diagnostic apparatus according to claim 5,
wherein the dialogue display control unit (13) is configured to display, on the display unit (5), the dialogue (D) in which the examination parts corresponding to the plurality of collation patterns are displayed so as to be arranged in descending order of the degrees of confidence calculated for the plurality of collation patterns.

8. The ultrasound diagnostic apparatus according to claim 5,
wherein the dialogue display control unit (13) is configured to display, on the display unit (5), the dialogue (D) in which the examination parts corresponding to the collation patterns, for which the degrees of confidence have been calculated, and values of the degrees of confidence are displayed.

9. The ultrasound diagnostic apparatus according to claim 5,
wherein the dialogue display control unit (13) is configured to display, on the display unit (5), the dialogue (D) in which the examination part that has been examined is not displayed and an examination part that has not been examined is displayed.

10. The ultrasound diagnostic apparatus according to any one of claims 2 to 9, further comprising:
a part confirmation unit (14) configured to confirm that the examination part with the degree of confidence greater than the predetermined threshold value is the imaging part of the subject in a case in which only one of the calculated degrees of confidence is greater than the predetermined threshold value, and to confirm the imaging part of the subject on the basis of a check result of the user through the dialogue (D) in cases other than the case in which only one of the calculated degrees of confidence is greater than the predetermined threshold value.

11. The ultrasound diagnostic apparatus according to claim 10, further comprising:
an imaging condition setting unit (11) configured to set imaging conditions corresponding to the imaging part confirmed by the part confirmation unit (14),
wherein the imaging unit (3) is configured to generate the B-mode image according to the imaging conditions set by the imaging condition setting unit.

12. A method for controlling an ultrasound diagnostic apparatus, the method comprising:
a step of transmitting an ultrasound beam to and receiving an ultrasound echo from an imaging part of a subject using an ultrasound probe (1) and converting a received signal output from the ultrasound probe into an image to generate a B-mode image of the subject;
a step of reading a plurality of collation patterns that are stored in advance, collating the B-mode image with each of the plurality of read collation patterns to perform part recognition, and calculating degrees of confidence for examination parts corresponding to each of the read collation patterns; and
determining whether to display, on a display unit (5), a dialogue (D) for making a user check which of a plurality of examination parts, for which the degrees of confidence have been calculated, is an imaging part of the subject on the basis of the degrees of confidence and displaying the dialogue on the display unit on the basis of a determination result;
**characterized by** displaying the dialogue (D) on the display unit (5) in a case in which the degree of confidence calculated for the collation pattern corresponding to an examination part that has been examined among the calculated degrees of confidence is greater than a predetermined threshold value.

## Patentansprüche

1. Ultraschalldiagnosevorrichtung, umfassend:
eine Ultraschallsonde (1);
eine Bildgebungseinheit (3), konfiguriert zum Senden eines Ultraschallstrahls zu einem Abbildungsteil eines Subjekts und zum Empfangen eines Ultraschallechos von dem Abbildungsteil unter Verwendung der Ultraschallsonde, und zum Umwandeln eines von der Ultraschallsonde ausgegebenen empfangenen Signals in ein Bild, um ein B-Modus-Bild des Subjekts zu erzeugen;
eine Anzeigeeinheit (5);
einen Kollationsmusterspeicher (16), konfiguriert zum Vorab-Speichern einer Mehrzahl von Kollationsmustern entsprechend einer Mehrzahl von Untersuchungsteilen,
eine Vertrauensmaß-Berechnungseinheit (12), konfiguriert zum Lesen der mehreren Kollationsmuster aus dem Kollationsmusterspeicher, um das von der Bildgebungseinheit (3) erzeugte B-Modus-Bild mit jedem der mehreren gelesenen Kollationsmuster zu vergleichen, um eine Teileerkennung auszuführen, und zum Berechnen von Vertrauensmaßen für die Untersuchungsteile entsprechend jedem der gelesenen Kollationsmuster; und
eine Dialog-Anzeigesteuereinheit (13), konfiguriert zum Bestimmen, ob auf der Anzeigeeinheit (5) ein Dialog (D) anzuzeigen ist, um einen Benutzer zu veranlassen, zu prüfen, welches der mehreren Untersuchungsteile, für die die Vertrauensmaße berechnet wurden, sich in dem Abbildungsteil des Subjekts befindet, auf der Grundlage der Vertrauensmaße, und zum Anzeigen des Dialogs auf der Anzeigeeinheit auf der Grundlage des Bestimmungsergebnisses;
**dadurch gekennzeichnet, dass** für den Fall, dass das Vertrauensmaß, das für das Kollationsmuster entsprechend einem untersuchten Untersuchungsteil berechnet wurde, unter den berechneten Vertrauensmaßen größer ist als ein vorbestimmter Schwellenwert, die Dialog-Anzeigesteuereinheit (13) konfiguriert ist zum Anzeigen des Dialogs (D) auf der Anzeigeeinheit (5).

2. Ultraschalldiagnosevorrichtung nach Anspruch 1,
bei der für den Fall, dass nur eines der berechneten Vertrauensmaße größer als der vorbestimmte Schwellenwert ist, die Dialog-Anzeigesteuereinheit (13) konfiguriert ist, um den Dialog (D) auf der Anzeigeeinheit (5) nicht anzuzeigen, und
in Fällen verschieden von dem Fall, dass nur eines der berechneten Vertrauensmaße größer als der vorbestimmte Schwellenwert ist, die Dialog-Anzeigesteuereinheit konfiguriert ist zum Anzeigen des Dialogs auf der Anzeigeeinheit.

3. Ultraschalldiagnosevorrichtung nach Anspruch 2,
bei der für den Fall, dass sämtliche der berechneten Vertrauensmaße nicht größer als der vorbestimmte Schwellenwert sind, die Dialog-Anzeigesteuereinheit (13) konfiguriert ist zum Anzeigen des Dialogs auf der Anzeigeeinheit (5).

4. Ultraschalldiagnosevorrichtung nach Anspruch 2,
bei der für den Fall, dass eine Mehrzahl von Vertrauensmaßen unter den berechneten Vertrauensmaßen größer ist als der vorbestimmte Schwellenwert, die Dialog-Anzeigesteuereinheit (13) konfiguriert ist zum Anzeigen des Dialogs (D) auf der Anzeigeeinheit (5).

5. Ultraschalldiagnosevorrichtung nach einem der Ansprüche 1 bis 4,
bei der die Dialog-Anzeigesteuereinheit (13) konfiguriert ist, um auf der Anzeigeeinheit (5) den Dialog (D) anzuzeigen, der gemäß den berechneten Vertrauensmaßen gebildet ist.

6. Ultraschalldiagnosevorrichtung nach Anspruch 5,
bei der die Dialog-Anzeigesteuereinheit (13) konfiguriert ist, um auf der Anzeigeeinheit (5) den Dialog (D) anzuzeigen, in welchem ein Untersuchungsteil, das dem Kollationsmuster mit einem höchsten Vertrauensmaß unter den Vertrauensmaßen, die für jedes der mehreren Kollationsmuster berechnet wurden, entspricht, derart angezeigt wird, dass er größer ist als andere Untersuchungsteile.

7. Ultraschalldiagnosevorrichtung nach Anspruch 5,
bei der die Dialog-Anzeigesteuereinheit (13) konfiguriert ist, um auf der Anzeigeeinheit (5) den Dialog (D) anzuzeigen, in welchem die Untersuchungsteile entsprechend den mehreren Kollationsmustern derart angezeigt sind, dass sie in absteigender Reihenfolge der für die mehreren Kollationsmuster berechneten Vertrauensmaße angeordnet sind.

8. Ultraschalldiagnosevorrichtung nach Anspruch 5,
bei der die Dialog-Anzeigesteuereinheit (13) konfiguriert ist, um auf der Anzeigeeinheit (5) den Dialog (D) anzuzeigen, in welchem die Untersuchungsteile entsprechend den Kollationsmustern, für die die Vertrauensmaße berechnet wurden, und Werte der Vertrauensmaße anzuzeigen.

9. Ultraschalldiagnosevorrichtung nach Anspruch 5,
bei der die Dialog-Anzeigesteuereinheit (13) konfiguriert ist, um auf der Anzeigeeinheit (5) den Dialog (D) anzuzeigen, in welchem das Untersuchungsteil, welches untersucht wurde, nicht angezeigt wird, und ein Untersuchungsteil, das nicht untersucht wurde, angezeigt wird.

10. Ultraschalldiagnosevorrichtung nach einem der Ansprüche 2 bis 9, weiterhin umfassend:
eine Teilebestätigungseinheit (14), konfiguriert zum Bestätigen, dass das Untersuchungsteil mit dem Vertrauensmaß größer als der vorbestimmte Schwellenwert das Abbildungsteil des Subjekts ist, falls nur eines der berechneten Vertrauensmaße größer ist als der vorbestimmte Schwellenwert, und um das Abbildungsteil des Subjekts zu bestätigen auf der Grundlage eines Prüfergebnisses seitens des Benutzers über den Dialog (D) in solchen Fällen verschieden von dem Fall, dass nur eines der berechneten Vertrauensmaße größer als der vorbestimmte Schwellenwert ist.

11. Ultraschalldiagnosevorrichtung nach Anspruch 10, weiterhin umfassend:
eine Abbildungsbedingungs-Einstelleinheit (11), konfiguriert zum Einstellen von Abbildungsbedingungen entsprechend dem Abbildungsteil, der von der Teilebestätigungseinheit (14) bestätigt wurde,
wobei die Bildgebungseinheit (3) konfiguriert ist zum Erzeugen des B-Modus-Bilds nach Maßgabe der Bildgebungsbedingungen, die von der Abbildungsbedingungs-Einstelleinheit eingestellt wurden.

12. Verfahren zum Steuern einer Ultraschalldiagnosevorrichtung, umfassend:
einen Schritt des Sendens eines Ultraschallstrahls zu und zum Empfangen eines Ultraschallechos von einem Abbildungsteil eines Subjekts unter Verwendung einer Ultraschallsonde (1) und des Umwandelns eines von der Ultraschallsonde ausgegebenen empfangenen Signals in ein Bild, um ein B-Modus-Bild des Subjekts zu erzeugen,
einen Schritt des Lesens einer Mehrzahl von Kollationsmustern, die vorab gespeichert wurden, des Vergleichens des B-Modus-Bilds mit jedem der mehreren gelesenen Kollationsmuster, um eine Teileerkennung durchzuführen, und des Berechnens von Vertrauensmaßen für Untersuchungsteile entsprechend jedem der gelesenen Kollationsmuster; und
Bestimmen, ob auf einer Anzeigeeinheit (5) ein Dialog (D) anzuzeigen ist, um einen Benutzer aufzufordern, zu prüfen, welches einer Mehrzahl von Untersuchungsteilen, für die die Vertrauensmaße berechnet wurden, ein Abbildungsteil des Subjekts ist, auf der Grundlage des Vertrauensmaßes, und Anzeigen des Dialogs auf der Anzeigeeinheit auf der Grundlage eines Bestimmungsergebnisses,
**dadurch gekennzeichnet, dass** der Dialog (D) auf der Anzeigeeinheit (5) für den Fall angezeigt wird, in welchem das Vertrauensmaß, welches für das Kollationsmuster entsprechend einem untersuchten Untersuchungsteil berechnet wurde, unter den berechneten Vertrauensmaßen größer ist als ein vorbestimmter Schwellenwert.

## Revendications

1. Appareil de diagnostic à ultrasons, comprenant :
une sonde ultrasonore (1) ;
une unité d'imagerie (3) configurée pour transmettre un faisceau ultrasonore et recevoir un écho ultrasonore en direction et en provenance d'une partie d'imagerie d'un sujet à l'aide de la sonde ultrasonore, et convertir un signal reçu produit à partir de la sonde ultrasonore en une image pour produire une image en mode B du sujet ;
une unité d'affichage (5) ;
une mémoire de motifs de collationnement (16) configurée pour stocker une pluralité de motifs de collationnement correspondant à une pluralité de parties d'examen du sujet à l'avance ;
une unité de calcul de degré de confiance (12) configurée pour lire la pluralité de motifs de collationnement dans la mémoire de motifs de collationnement, pour collationner l'image en mode B générée par l'unité d'imagerie (3) avec chaque motif de la pluralité de motifs de collationnement lus pour réaliser une reconnaissance de partie, et pour calculer des degrés de confiance pour les parties d'examen correspondant à chacun des motifs de collationnement lus, et
une unité de commande d'affichage de dialogue (13) configurée pour déterminer s'il faut afficher ou non, sur l'unité d'affichage (5), un dialogue (D) pour faire en sorte qu'un utilisateur vérifie quelle partie parmi la pluralité de parties d'examen, pour laquelle les degrés de confiance ont été calculés, est la partie d'imagerie du sujet sur la base des degrés de confiance, et afficher le dialogue sur l'unité d'affichage sur la base d'un résultat de détermination ;
**caractérisé en ce que** dans un cas où le degré de confiance calculé pour le motif de collationnement correspondant à une partie d'examen ayant été examinée parmi les degrés calculés de confiance est supérieur à une valeur seuil prédéterminée, l'unité de commande d'affichage de dialogue (13) est configurée pour afficher le dialogue (D) sur l'unité d'affichage (5).

2. Appareil de diagnostic à ultrasons selon la revendication 1,
dans lequel dans un cas où un seul des degrés calculés de confiance est supérieur à une valeur seuil prédéterminée, l'unité de commande d'affichage de dialogue (13) est configurée pour ne pas afficher le dialogue (D) sur l'unité d'affichage (5), et
dans des cas autres que le cas où un seul des degrés calculés de confiance est supérieur à la valeur seuil prédéterminée, l'unité de commande d'affichage de dialogue est configurée pour afficher le dialogue sur l'unité d'affichage.

3. Appareil de diagnostic à ultrasons selon la revendication 2,
dans lequel dans un cas où tous les degrés calculés de confiance ne sont pas supérieurs à la valeur seuil prédéterminée, l'unité de commande d'affichage de dialogue (13) est configurée pour afficher le dialogue (D) sur l'unité d'affichage (5).

4. Appareil de diagnostic à ultrasons selon la revendication 2,
dans lequel dans un cas où une pluralité de degrés de confiance parmi les degrés calculés de confiance sont supérieurs à la valeur seuil prédéterminée, l'unité de commande d'affichage de dialogue (13) est configurée pour afficher le dialogue (D) sur l'unité d'affichage (5).

5. Appareil de diagnostic à ultrasons selon l'une quelconque des revendications 1 à 4,
dans lequel l'unité de commande d'affichage de dialogue (13) est configurée pour afficher le dialogue (D) formé conformément aux degrés calculés de confiance sur l'unité d'affichage (5).

6. Appareil de diagnostic à ultrasons selon la revendication 5,
dans lequel l'unité de commande d'affichage de dialogue (13) est configurée pour afficher, sur l'unité d'affichage (5), le dialogue (D), où une partie d'examen correspondant au motif de collationnement avec un degré le plus élevé de confiance parmi les degrés de confiance calculés pour chaque motif de la pluralité de motifs de collationnement, est affiché de manière à être plus grand que d'autres parties d'examen.

7. Appareil de diagnostic à ultrasons selon la revendication 5,
dans lequel l'unité de commande d'affichage de dialogue (13) est configurée pour afficher, sur l'unité d'affichage (5), le dialogue (D), où les parties d'examen correspondant à la pluralité de motifs de collationnement sont affichées de manière à être agencées en ordre décroissant des degrés de confiance calculés pour la pluralité de motifs de collationnement.

8. Appareil de diagnostic à ultrasons selon la revendication 5,
dans lequel l'unité de commande d'affichage de dialogue (13) est configurée pour afficher, sur l'unité d'affichage (5), le dialogue (D), où les parties d'examen correspondant aux motifs de collationnement, pour lesquels les degrés de confiance ont été calculés, et des valeurs des degrés de confiance sont affichées.

9. Appareil de diagnostic à ultrasons selon la revendication 5,
dans lequel l'unité de commande d'affichage de dialogue (13) est configurée pour afficher, sur l'unité d'affichage (5), le dialogue (D), où la partie d'examen ayant été examinée n'est pas affichée et une partie d'examen n'ayant pas été examinée est affichée.

10. Appareil de diagnostic à ultrasons selon l'une quelconque des revendications 2 à 9, comprenant en outre :
une unité de confirmation de partie (14) configurée pour confirmer que la partie d'examen avec le degré de confiance supérieur à la valeur seuil prédéterminée est la partie d'imagerie du sujet dans le cas où un seul des degrés calculés de confiance est supérieur à la valeur seuil prédéterminée, et confirmer la partie d'imagerie du sujet sur la base d'un résultat de vérification de l'utilisateur par le biais du dialogue (D) dans des cas autres que le cas où un seul des degrés calculés de confiance est supérieur à la valeur seuil prédéterminée.

11. Appareil de diagnostic à ultrasons selon la revendication 10, comprenant en outre :
une unité de réglage de condition d'imagerie (11), configurée pour régler des conditions d'imagerie correspondant à la partie d'imagerie confirmée par l'unité de confirmation de partie (14), et
dans lequel l'unité d'imagerie (3) est configurée pour générer l'image en mode B conformément aux conditions d'imagerie réglées par l'unité de réglage de condition d'imagerie.

12. Procédé de commande d'un appareil de diagnostic à ultrasons, le procédé comprenant les étapes suivantes :
une étape pour transmettre un faisceau ultrasonore et recevoir un écho ultrasonore en direction et en provenance d'une partie d'imagerie d'un sujet à l'aide d'une sonde ultrasonore (1), et convertir un signal reçu produit à partir de la sonde ultrasonore en une image pour produire une image en mode B du sujet ;
une étape pour lire une pluralité de motifs de collationnement stockés à l'avance, collationner l'image en mode B avec chaque motif de la pluralité de motifs de collationnement lus pour réaliser une reconnaissance de partie, et pour calculer des degrés de confiance pour les parties d'examen correspondant à chacun des motifs de collationnement lus, et
une étape pour déterminer s'il faut afficher ou non, sur l'unité d'affichage (5), un dialogue (D) pour faire en sorte qu'un utilisateur vérifie quelle partie parmi la pluralité de parties d'examen, pour laquelle les degrés de confiance ont été calculés, est la partie d'imagerie du sujet sur la base des degrés de confiance, et afficher le dialogue sur l'unité d'affichage sur la base d'un résultat de détermination ;
**caractérisé par** l'affichage du dialogue (D) sur l'unité d'affichage (5) dans un cas où le degré de confiance calculé pour le motif de collationnement correspondant à une partie d'examen ayant été examinée parmi les degrés calculés de confiance est supérieur à une valeur seuil prédéterminée.
